# EUROPEAN PATENT SPECIFICATION

(11) **EP 2 828 375 B1**
(45) Date of publication and mention of the grant of the patent: **22.11.2017**
(21) Application number: 13708357.2
(22) Date of filing: 21.01.2013
(51) Int. Cl.: C12N 1/20, C12P 13/00, A61K 35/74, C12R 1/01, C12R 1/24

(54) **GABA-PRODUCING CULTURABLE BACTERIA DERIVED FROM THE HUMAN GASTROINTESTINAL TRACT**
GABA-ERZEUGENDE, AUS DEM MENSCHLICHEN MAGEN-DARM-TRAKT STAMMENDE KULTIVIERBARE BAKTERIE
BACTÉRIES POUVANT ÊTRE MISES EN CULTURE, PRODUISANT DE L'ACIDE GABA ET DÉRIVÉES DU TRACTUS GASTRO-INTESTINAL

(30) Priority: 19.01.2012 EP 12151768
(43) Date of publication of application: 28.01.2015
(73) Proprietor: University College Cork-National University of Ireland, Cork, Cork (IE); Agriculture and Food Development Authority (TEAGASC), Carlow (IE)
(72) Inventor: STANTON, Catherine, Kilworth Co. Cork (IE); ROSS, Paul, Kilworth Co. Cork (IE); CRYAN, John, Cork Co. Cork (IE); DINAN, Ted, Cork Co. Cork (IE)
(74) Representative: Purdy, Hugh Barry
(86) International application number: PCT/EP2013/051065
(87) International publication number: WO 2013/107913

(56) References cited:
- WO-A1-2005/038008
- SIRAGUSA S ET AL: "Synthesis of gamma-aminobutyric acid by lactic acid bacteria isolated from a variety of Italian cheeses", APPLIED AND ENVIRONMENTAL MICROBIOLOGY, vol. 73, no. 22, November 2007 (2007-11), pages 7283-7290, XP002677194, ISSN: 0099-2240
- PARK ET AL: "Cloning, sequencing and expression of a novel glutamate decarboxylase gene from a newly isolated lactic acid bacterium, Lactobacillus brevis OPK-3", BIORESOURCE TECHNOLOGY, ELSEVIER BV, GB, vol. 98, no. 2, 1 January 2007 (2007-01-01), pages 312-319, XP005645219, ISSN: 0960-8524, DOI: 10.1016/J.BIORTECH.2006.01.004
- HAIXING LI ET AL: "Lactic acid bacterial cell factories for gamma-aminobutyric acid", AMINO ACIDS ; THE FORUM FOR AMINO ACID AND PROTEIN RESEARCH, SPRINGER-VERLAG, VI, vol. 39, no. 5, 3 April 2010 (2010-04-03), pages 1107-1116, XP019856927, ISSN: 1438-2199, DOI: 10.1007/S00726-010-0582-7
- Bo Jiang, Yuanxin Fu and Tao Zhang: "Gamma-Aminobutyric Acid"; "Chapter 9" In: Yoshinori Mine, Eunice Li-Chan and Bo Jiang: "Bioactive Proteins and Peptides as Functional Foods and Nutraceuticals", July 2010 (2010-07), Blackwell Publishing Ltd. and Institute of Food Technologists, XP002677195, ISBN: 978-0-8138-1311-0 pages 121-133, the whole document
- LYTE MARK: "Probiotics function mechanistically as delivery vehicles for neuroactive compounds: Microbial endocrinology in the design and use of probiotics", BIOESSAYS, vol. 33, no. 8, August 2011 (2011-08), pages 574-581, XP002677196, ISSN: 0265-9247
- SHIN HO-YOUNG ET AL: "Purification and characterization of alpha-L-arabinopyranosidase and alpha-L-arabinofuranosidase from Bifidobacterium breve K-110, a human intestinal anaerobic bacterium metabolizing ginsenoside Rb2 and Rc.", APPLIED AND ENVIRONMENTAL MICROBIOLOGY, vol. 69, no. 12, December 2003 (2003-12), pages 7116-7123, XP002677197, ISSN: 0099-2240
- KI-BUM PARK ET AL: "Expression of Rice Glutamate Decarboxylase in Bifidobacterium Longum Enhances [gamma]-Aminobutyric Acid Production", BIOTECHNOLOGY LETTERS, SPRINGER NETHERLANDS, DORDRECHT, vol. 27, no. 21, 1 November 2005 (2005-11-01), pages 1681-1684, XP019231024, ISSN: 1573-6776, DOI: 10.1007/S10529-005-2730-9
- E. BARRETT ET AL: "[gamma]-Aminobutyric acid production by culturable bacteria from the human intestine", JOURNAL OF APPLIED MICROBIOLOGY, vol. 113, no. 2, 15 August 2012 (2012-08-15), pages 411-417, XP55066783, ISSN: 1364-5072, DOI: 10.1111/j.1365-2672.2012.05344.x

## Description

### Introduction

γ-Amino butyric acid (GABA) is a major inhibitory neurotransmitter of the vertebrate central nervous system. It is involved in the regulation of cardiovascular conditions such as blood pressure and heart rate, and plays a role in the sensation of pain and anxiety. A number of further potential health benefits of GABA have been described, including control of growth hormone secretion, protective effect against glycerol-induced acute renal failure in rats and anti-proliferative activity (a reduction of the induced migratory activity of SW480 colon carcinoma cell.

Interest in the potential role of GABA as an anti-hypertensive dietary component has recently increased in Japan. This was due in particular to the high sodium intake in the diet in that country, where the daily intake of salt was estimated to be 11.2 g/d in 2003. Interestingly, foods enriched with GABA have been defined as 'foods for specified health use (FOSHU) in Japan. This led to increased research into the development of fermented products containing GABA. A fermented milk product containing GABA was developed which was shown to have a blood pressure-lowering effect in spontaneously hypertensive rats and in mildly hypertensive people. Recent years have also witnessed an increase in the development of additional GABA-enriched fermented dairy, soybean, kimchi and juice products, which could be used as potential GABA delivery vehicles.

In addition to heightened interest in the possible physiological effects following the consumption of GABA-enriched fermented food, there was a concomitant surge in the isolation of novel fermented food-derived or dairy-starter cultures with the ability to produce GABA. Lactic acid bacteria (LAB) from food sources have been demonstrated to have the ability to produce γ-amino butyric acid (GABA). GABA is produced primarily from the irreversible α-decarboxylation of L-glutamate by the enzyme glutamate decarboxylase (GAD), a pyridoxal 5'-phosphate (PLP)-dependant enzyme. It has been found in animals, higher plants and bacteria, where it plays a role in acid resistance. Many studies have reported the presence of a gad gene in LAB and indeed, GABA has been produced by cheese starters during cheese ripening. It is clear that one approach to increasing GABA levels in humans is by consuming GABA-enriched food products. However, when taken as a supplement, the recommended dosage of GABA is 1-2g per day, which is a lot of active agent to incorporate into a single food product such as a yoghurt or a drink. Lyte et al describe GABA-producing lactic acid bacteria of the genera Lactobacillus and Bifidobacterium (Mark Lyte, Bioassays 33: 574-581; 2011; Wiley Periodicals Inc).

It is an object of the invention to overcome at least one of the above-referenced problems.

### Statements of Invention

The Applicant has realised that another potential way of increasing GABA levels in the gut is by harnessing the production ability of intestinal microbiota or ingested probiotic bacteria, that could use dietary glutamate to generate GABA. To date, human GIT-derived bacteria having the ability to produce GABA have not been described. Set with the objective of identifying such bacteria, the Applicant has successfully discovered three strains of bacteria that are ideally capable of being cultured, that are derived from the human *neonatal* gastrointestinal tract (GIT), and that are capable of efficiently producing γ-aminobutyric acid (GABA) from a source of glutamate. The bacteria are *Lactobacillus brevis* DPC6108, *Bifidobacterium dentium* DPC6333, and *Bifidobacterium adolescentis* DPC6044. The Applicant has also discovered that the strains *Bifidobacterium infantis* UCC35624, which was originally derived from the human GIT, and the strain *Bifidobacteriumm dentium* deposited at the NCFB (National Collection of Food Bacteria) *NCFB2243,* originally derived from dental caries, are efficient producers of GABA from a source of glutamate.

In particular, the strain *Lactobacillus brevis* DPC6108 has been found to demonstrate a highly efficient conversion of monosodium glutamate to GABA in an *in-vitro* conversion test (100% conversion of 10 and 20mg/ml MSG- Table 1). In addition, *in-vivo* behavioural tests including the Open Field Test and Forced Swim Test indicate that administration of *Lactobacillus brevis* DPC6108 to rats confers clinically significant anxiolytic effects compared to a placebo group (see end of results section).

Thus, in a first aspect, the invention provides an isolated bacteria *Lactobacillus brevis* DPC6108,
**t**he bacteria being characterised in that is it ideally culturable, is preferably derived from a mammalian gastrointestinal tract (especially the human neonatal GIT), and has the ability to produce γ-aminobutyric acid (GABA). Typically, the isolated bacteria can be derived from a human neonatal faeces sample. Ideally, the isolated bacteria is capable of efficient conversion of MSG to GABA in an *in-vitro* conversion test, for example at least 50%, 60%, 70%, 80%, 90% or 100% conversion of MSG to GABA. Suitably, the isolated bacteria of the invention is a probiotic bacteria.

### Lb. brevis DPC6108 strain

In a preferred embodiment, the invention relates to an isolated bacteria *Lactobacillus brevis* (DPC6108) deposited with the National Collection of Industrial and Marine Bacteria Limited (NCIMB) on 28 November 2011 and accorded the accession number NCIMB 41903, the bacteria being characterised in that is it culturable, and is derived from a human neonatal gastrointestinal tract.

A deposit of *Lb. brevis* DPC6108 was made at the National Collection of Industrial and Marine Bacteria Limited (NCIMB) on 28 November 2011 by the Agricultural and Food Development Authority (Teagasc) of Oak Park, Carlow, Ireland and accorded the accession number NCIMB 41903.

Typically, the isolated *Lactobacillus brevis* (DPC6108) strain of the invention, is capable of 100% conversion of monosodium glutamate (MSG) to γ-aminobutyric acid (GABA) in an *in-vitro* conversion test described below

Suitably, the isolated *Lactobacillus brevis* (DPC6108) strain of the invention, is derived from a human neonatal faecal sample.

Typically, the isolated *Lactobacillus brevis* (DPC6108) strain comprises a 16S rRNA sequence of SEQ ID NO: 1

In one embodiment, the isolated *Lactobacillus brevis* (DPC6108) strain of the invention, is provided in the form of live cells, dead cells, cellular components, cell extracts, or cell lysates.

### B. dentium DPC6333 strain

A deposit of *B. dentium* DPC6333 was made at the National Collection of Industrial and Marine Bacteria Limited (NCIMB) on 28 November 2011 by the Agricultural and Food Development Authority (Teagasc) of Oak Park, Carlow, Ireland and accorded the accession number NCIMB 41904.

Typically, the isolated *B. dentium* DPC6333 strain is capable of 50% conversion of monosodium glutamate (MSG) to γ-aminobutyric acid (GABA) in the *in-vitro* conversion test described below

Suitably, the isolated *B. dentium* DPC6333 strain is derived from a human neonatal faecal sample.

In one embodiment, the isolated *B. dentium* DPC6333 strain is provided in the form of live cells, dead cells, cellular components, cell extracts, or cell lysates.

### B. adolescentis DPC6044

A deposit of *B. adolescentis* DPC6044 was made at the National Collection of Industrial and Marine Bacteria Limited (NCIMB) on 2 December 2011 by the Agricultural and Food Development Authority (Teagasc) of Oak Park, Carlow, Ireland and accorded the accession number NCIMB 41908.

Typically, the isolated *B. adolescentis* DPC6044 strain is capable of at least 20% conversion of monosodium glutamate (MSG) to γ-aminobutyric acid (GABA) in the *in-vitro* conversion test described below

Suitably, the isolated *B. adolescentis* DPC6044 strain is derived from a human neonatal faecal sample.

In one embodiment, the isolated *B. adolescentis* DPC6044 strainis provided in the form of live cells, dead cells, cellular components, cell extracts, or cell lysates.

### B. infantis UCC35624

A deposit of *B. infantis* UCC35624 was made at the National Collection of Industrial and Marine Bacteria Limited (NCIMB) on 13 January 1999 and accorded the accession number NCIMB 41003. The characteristics of the bacteria are described in US patent publication No: US2010/0112003A1 (the complete contents of which are incorporated herein by reference).

Typically, the isolated *B. infantis* UCC35624 strain is capable of at least 25% or 35% conversion of monosodium glutamate (MSG) to γ-aminobutyric acid (GABA) in the *in-vitro* conversion test described below

Suitably, the isolated *B. infantis* UCC35624 strain is derived from a human neonatal faecal sample.

In one embodiment, the isolated *B. infantis* UCC35624 strain is provided in the form of live cells, dead cells, cellular components, cell extracts, or cell lysates.

### Compositions

The invention also relates to a composition comprising an isolated bacteria of the invention, preferably *Lb. brevis* DPC6108 strain.

Typically, the composition is provided in the form of a product formulated for human ingestion, for example, a food product, a drink, a food supplement, or a medicament.

Suitably, the composition of the invention further includes at least one bacterial culture selected from the group consisting of:
- *Bifidobacterium dentium* (DPC6333) NCIMB 41904 deposited on 28 November 2011;
- *Bifidobacterioum adolescentis* (DPC6044) NCIMB 41908 deposited on 2 December 2011;
- *Bifidobacterium infantis* (UCC35624) NCIMB 41003 deposited on 13 January 1999; and
- *Bifidobacterium dentium NCFB2243*

Typically, the composition comprises a source of glutamate, for example a glutamate salt such as, for example, monosodium glutamate.

### Medical Indications

The invention also relates to an isolated bacteria of the invention, or a composition of the invention, for use as a medicament.

The invention also relates to an isolated bacteria of the invention, or a composition of the invention, **for** use in the regulation or treatment of cardiovascular function, growth hormone secretion, mood disturbance, anxiety, stress, pain, renal failure, or proliferative conditions .

Typically, the composition is administered to an individual orally. Upon ingestion, the isolated bacteria of the composition that has been administered will form part of the individuals microbiota.

Suitably, the composition includes free glutamate.

In a preferred embodiment, the indication is anxiety.

In another embodiment, the indication is dysregulated blood pressure.

In another embodiment, the indication is mood disturbance.

In another embodiment, the indication is a proliferative disorder.

In a particularly preferred embodiment, the invention relates to a composition for **use** in the regulation or treatment of anxiety, in which the composition comprises an isolated bacteria *Lactobacillus brevis* (DPC6108) deposited with the National Collection of Industrial and Marine Bacteria Limited (NCIMB) on 28 November 2011 and accorded the accession number NCIMB 41903, the bacteria being characterised in that is it culturable, is derived from a human neonatal gastrointestinal tract, and is optionally capable of 70%, 80%, 90% or 100% conversion of monosodium glutamate (MSG) to γ-aminobutyric acid (GABA) *in-vitro..*

In another embodiment, the invention relates to a composition for **use** as a medicament, in which the composition comprises an isolated bacteria *Lactobacillus brevis* (DPC6108) deposited with the National Collection of Industrial and Marine Bacteria Limited (NCIMB) on 28 November 2011 and accorded the accession number NCIMB 41903, the bacteria being characterised in that is it culturable, is derived from a human neonatal gastrointestinal tract, and is optionally capable of 70%, 80%, 90% or 100% conversion of monosodium glutamate (MSG) to γ-aminobutyric acid (GABA) *in-vitro..*

In another embodiment, the invention relates to a composition for **use** in producing γ-aminobutyric acid, in which the composition comprises an isolated bacteria *Lactobacillus brevis* (DPC6108) deposited with the National Collection of Industrial and Marine Bacteria Limited (NCIMB) on 28 November 2011 and accorded the accession number NCIMB 41903, the bacteria being characterised in that is it culturable, is derived from a human gastrointestinal tract, and is optionally capable of 70%, 80%, 90% or 100% conversion of monosodium glutamate (MSG) to γ-aminobutyric acid (GABA) *in-vitro.*

The invention also relates to a method of regulation or treatment of cardiovascular function, growth hormone secretion, mood disturbance, anxiety, stress, pain, renal failure, or proliferative conditions in a mammal, typically a human,, comprising a step of administering to the mammal an isolated bacteria of the invention or a composition of the invention.

The bacteria referenced above are probiotic bacteria which should be understood to mean live microorganisms which when administered in adequate amounts confer a health benefit on the host.

### Brief Description of the Figure

**Figure 1****:** Growth and GABA production by Lactobacillus brevis DPC6108 in mMRS in the presence of 30 mg/ml monosodium glutamate. □ Log CFU/ml; ▲ µg/ml GABA.
**Figure 2****:** GABA production by culturable gut bacteria and *L*. *brevis* DPC6108 in a pH controlled faecal fermentation containing 30 mg/ml monosodium glutamate. Subject 1 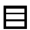 Subject 2 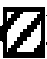 Subject 3 □ plus ∼10⁸ *L*. *brevis* DPC6108 ■

### Detailed Description of the Invention

A deposit of *Lb. brevis* DPC6108 was made at the National Collection of Industrial and Marine Bacteria Limited (NCIMB) on 28 November 2011 and accorded the accession number NCIMB 41903. The strain of bacteria was isolated from the neonatal faecal sample using the techniques described in Wall et al, FEMS Microbiol Ecol 59 (2007) 127-137 (the complete contents of which are incorporated herein by reference).

A deposit of *B. dentium* DPC6333 was made at the National Collection of Industrial and Marine Bacteria Limited (NCIMB) on 28 November 2011 and accorded the accession number NCIMB 41904. The strain of bacteria was isolated from the neonatal faecal sample using the techniques described in Barrett et al, Applied and Environmental Microbiology, Apr. 2007, p2333-2337 (the complete contents of which are incorporated herein by reference).

A deposit of *B. adolescentis* DPC6044 was made at the National Collection of Industrial and Marine Bacteria Limited (NCIMB) on 2 December 2011 and accorded the accession number NCIMB 41908. The strain of bacteria was isolated from the neonatal faecal sample using the techniques described in Rosberg-Cody et al, Applied and Environmental Microbiology, Aug. 2004, p4625-4641 (the complete contents of which are incorporated herein by reference).

A deposit of *B. infantis* UCC35624 was made at the National Collection of Industrial and Marine Bacteria Limited (NCIMB) on 13 January 1999 and accorded the accession number NCIMB 41003. The characteristics of the bacteria are described in US patent publication No: US2010/0112003A1 (the complete contents of which are incorporated herein by reference).

The molecule GABA has been implicated as a means of providing health benefits to humans, for example regulation of cardiovascular function, renal function, and growth hormone secretion, and treatment of anxiety, mood disturbance (i.e. bad mood), and proliferative disorders. The term "proliferative disorders" should be understood to mean cancer, tumours, and metastases. The invention therefore also relates to a method of improving health in an individual comprising a step of administering to the individual an isolated bacteria of the invention in which the bacterial culture once administered becomes part of the individuals intestinal microbiota and produces GABA in-vivo from dietary glutamate.

The term "culturable" as applied to the bacterial strains of the invention should be understood to mean that the strains are viable and can be grown and enumerated in a laboratory using a synthetic growth medium.

The term *"in-vitro* conversion test" as employed herein refers to the *Measurement of γ-Amino butyric acid* test described below in which the mMRS broth is supplemented with 10mg/ml MSG.

The five bacterial species described above have the ability to produce GABA from a source of glutamate *in-vitro* and ideally also *in-vivo.* The source of glutamate is ideally free glutamate (i.e. glutamate that is not locked up in a molcule such as a protein or peptide) for example a glutamate salt such as monosodium glutamate. The bacterial species described above ideally have the ability to produce GABA from a source of glutamate *in-vivo,* ideally *in-vivo* in a human. This means that when a culture of the bacteria is ingested at least part of the culture takes up residence in the host intestinal microbiota and employs dietary glutamate (or glutamate derived from product in the diet) to generate GABA in the GIT.

The term "regulation of cardiovascular function" should be understood to mean modulation of dysregulated function. In the case of blood pressure, the term should be understood to mean helping return high blood pressure or low blood pressure to a normal healthy level. In the case of heart rate, the term should be understood to mean helping return a dysregulated heart rate back to a normal healthy rate.

The term "composition" as employd herein means a composition of matter including at least one bacterial culture as described above. Suitably, the composition is a product suitable for human or animal ingestion, for example a food product or drink, for example a dairy product including yoghurt or yoghurt drink, a milk product, butter, cheese or cheese product, a soy product, a snack bar or other type of food product. The composition may also take the form of a health supplement, for example a powder for mixing with a liquid to make for example a shake, or a capsule or pill. The composition may also take the form of a pharmaceutical composition comprising a bacterial culture as described above and a suitable pharmaceutical carrier, the details of which will be well known to those skilled in the art. The pharmaceutical composition may take the form of a tablet, a capsule, a liquid or a suspension.

The term "treatment" as applied to a pathology generally should be understood to mean reducing the symptoms of the pathology, curing the pathology, preventing the development of the pathology, or arresting the development of the pathology. In the case of mood disturbance, treatment should be understood to mean helping return the mood to a normal health mood, and also to prevent development of mood disturbance. In the case of anxiety, treatment should be understood to mean helping reduce or remove the feelings of anxiety, or prevent development of feelings of anxiety. In the case of pain, treatment should be understood to mean helping reduce or remove the pain, or prevent development of pain. In the case of stress, treatment refers to reducing or ameliorating the feelings of stress, or the prevention of the development of stress.

The term "for use as a medicament" as employed herein means that the composition is employed with a purpose of improving health, for example a food product that provides one or more health benefits or a pharmaceutical composition for a specific medical or veterinary indication, or a health supplement.

The composition, uses and methods of the invention are intended for use with mammals, but especially for use with humans and companion animals (for example dogs, cats and horses) and agricultural animals (for example bovine and porcine animals).

The bacterial cultures referred to above, or the derivatives thereof, may be in the form of live bacteria, dead bacteria or cellular components, or cell extracts or lysates thereof. Methods for preparing cellular components, cell extracts or cell lysates will be well known to those skilled in the art.

### Materials and Methods

### Maintenance and culture of bacterial strains:

The human-derived strains employed herein were maintained in the Teagasc Moorepark Food Research Centre culture collection. Strains of lactobacilli and bifidobacteria were cultured in MRS broth (Difco, Detroit, Mich.) supplemented with 0.05% (w/v) L-cysteine-hydrochloride (mMRS) (98% pure Sigma Chemical Co., St. Louis, Mo.) under anaerobic (anaerobic jars with Anaerocult®A gas packs; Merck, Darmstadt, Germany) conditions at 37°C. When solid medium was required, 1.5% (w/v) agar (Oxoid, Hampshire, UK) was added to the mMRS medium. Standard cultures were prepared by inoculation of 10 ml mMRS broth with 10 µl of a frozen stock (-80°C) followed by incubation at 37°C for 16-24 h. Strains were then subcultured in 10 ml mMRS broth for 16-24 h at 37°C prior to inoculation into the fermentation vessel.

### Measurement of γ-Amino butyric acid:

Prior to examination of the strains for GABA production, each strain was subcultured in mMRS. Strains were inoculated (1% (v/v)) anaerobically in mMRS broth supplemented with 30 mg/ml monosodium glutamic acid (MSG) (Sigma) at 37°C for 72 h. One ml of the culture broth was centrifuged at 14,000 x g for 1 min and the level of GABA in the culture supernatant was determined by analyzing the free amino acid content with a Beckmann 6300 High Performance Amino Acid Analyzer as described below.

### Growth of Lactobacillus brevis DPC6108 in the presence of MSG:

The growth of Lactobacillus brevis DPC6108 was monitored in mMRS supplemented with MSG (30 mg/ml). The strain was inoculated from a fresh overnight culture to a final cell density of 10⁶ CFU/ml and the culture was grown anaerobically at 37°C. Bacterial growth (cell counts on mMRS agar, following serial dilution in maximum recovery diluent (MRD), at 37°C for 48 h), and conversion of MSG to GABA (determined by analyzing the free amino acid content with a Beckmann 6300 High Performance Amino Acid Analyzer as described below), were monitored in triplicate at regular time intervals over 55 h.

### γ-Amino butyric acid production by selected gut bacteria in faecal fermentations with pH control:

The faecal fermentation medium was prepared as previously described (Fooks and Gibson 2003), with a single modification which involved the addition of 30 mg/ml MSG. The medium was allowed to cool overnight at room temperature following sterilization and 160ml were transferred to a fermentation vessel and flushed with nitrogen for 30 min prior to inoculation. Fresh faecal samples were obtained from three healthy adults, who had not been prescribed antibiotics in the previous 3 months. However, levels of MSG intake in their diet in the days before sampling were unknown. A fresh faecal slurry (20% (w/v)) was prepared in anaerobic MRD containing 0.05% (w/v) cysteine. The anaerobic fermentation medium was inoculated with 40 ml of fresh faecal slurry in each fermentation vessel. To determine the effect of spiking the fermentation medium with GABA-producing human-derived bacteria, Lb. brevis DPC6108 was grown anaerobically at 37°C in mMRS supplemented with 30 mg/ml MSG for 48 h. Cells were harvested by centrifugation at 4,000 x g for 10 min, washed once with anaerobic MRD containing 0.05% (w/v) cysteine and inoculated into the faecal fermentation slurry to give an initial number of ∼10⁸ cells/ml in the fermentation vessel. Fermentations were conducted at 37°C under nitrogen, and maintained at pH 6.8 for 24 h. Samples were withdrawn at 0, 4, 9, 21 and 24 h for GABA analysis. Faecal fermentations were carried out in duplicate

### Amino acid analysis:

Samples were de-proteinised by mixing equal volumes of 24% (w/v) tri-chloroacetic acid (TCA) and sample. They were then allowed to stand for 10 min before centrifuging at 14,000 x g (Microcentaur, MSE, UK) for 10 min. Supernatants were removed and diluted with 0.2M sodium citrate buffer, pH 2.2 to yield approximately 250 nmol of each amino acid residue. Samples were then diluted 1 in 2 with the internal standard, norleucine, to give a final concentration of 125 nm/ml. Amino acids were quantified using a Jeol JLC-500/V amino acid analyzer (Jeol (UK) Ltd., Garden City, Herts, UK) fitted with a Jeol Na⁺ high performance cation exchange column.

### Animal Studies:

Probiotic: *Lactobacillus brevis* DPC 6108
Negative control (Placebo): 15% trehalose
Positive control: commercial GABA powder (around 2.6 mg/Kg body weight)
Method of delivering probiotic: freeze-dried powders and GABA are resuspended in the drinking water
Dose of probiotic: ∼1 × 10⁹ CFU per day
Duration: 5 weeks
Diet: standard chow diet - contains the substrate (glutamate) and co-factor (pyridoxal phosphate) in the concentration necessary for GABA production by the probiotic strain
Model: Sprague-Dawley rat
Number of animals: 10 per group
Caging: Group housing (5 per cage)

### Trial Design:

The rats are divided into three groups (A-C, *n* = 10) and subjected to the following dietary treatments daily:
- *Group A*: Placebo freeze-dried powder (15% trehalose in dH₂O).
- *Group B*: GABA powder (2.6 mg/Kg BW) in combination with placebo freeze-dried powder (15% trehalose in dH₂O).
- *Group C*: *Lactobacillus brevis* DPC 6108

### Administration of L. brevis DPC 6108

Week 0-end: *L. brevis* DPC 6108, placebo and GABA powder are resuspended in the drinking water every day. The probiotic at a concentration based on that each rat gets ∼10⁹ CFU per day (based on that rats drink approx. 10 mL/100g bodyweight) and the GABA at a concentration of 2.6 mg/Kg BW/day.

Parameters measured during the study:
- Weight (three times a week)
- Behavioral analysis: at week 4 (Open Field and Forced Swim Test) and week 5 (Colorectal Distension).

### Endpoint Analysis

- Glutamate/GABA ratio in plasma and colon
- Serum collection for glucose, insulin, growth hormone and corticosterone analyses
- Gene expression (receptors GABA_{A}, GABA_{B}) on specific brain regions)
- Neurotransmitters levels on specific brain regions (DOPA, 5-HT,NE)
- Cytokine profile of spleen
- qPCR for quantification of *Lactobacillus* sp. from faecal content
- Cholesterol levels in liver

### Results

### Screening of culturable intestinal lactobacilli and bifidobacteria for the production of γ-Amino butyric acid:

The details of strains with the ability to produce GABA from MSG are shown in Table 1. The conversion abilities of these strains varied when grown in different concentrations of MSG. Lb. brevis DPC6108 was capable of 100% conversion of 10 and 20 mg/ml MSG to 11.03 +/-0.31 and 20.47 +/- 0.23 mg/ml GABA in mMRS. Increasing the MSG concentrations to 30, 40 or 50 mg/ml resulted in decreased percentage conversions of 94.4% (28.02 +/- 0.23 mg/ml GABA), 75.98% (30.39 +/- 0.56 mg/ml GABA) and 64.64% (32.32 +/- 1.24 mg/ml GABA) respectively by Lb. brevis DPC6108 (Table 1). The three remaining GABA producing strains, B. dentium DPC6333, B. infantis UCC35624 and B. adolescentis DPC6044 also efficiently converted MSG to GABA (Table 1). Conversion efficiencies of MSG to GABA ranged from 22% (2.2 +/- 0.43 mg/ml GABA) to 60.9% (6.09 +/- 0.22 mg/ml GABA) and 15.86% (3.17 +/-0.39 mg/ml GABA) to 61.6% (12.32 +/- 0.69 mg/ml GABA) in 10 and 20 mg/ml MSG in mMRS, respectively, by the 4 bifidobacterial strains. Similar to Lb. brevis DPC6108, increasing the MSG concentrations to 30, 40 or 50 mg/ml resulted in decreased percentage conversions of MSG to GABA (Table 1). All of the remaining 86 Bifidobacterium and Lactobacillus strains assayed in this study grew in mMRS containing 30 mg/ml MSG, but they did not convert MSG to GABA at any significant level.

**TABLE 1**

| | | **GABA (mg/ml) converted from increasing concentrations of MSG (mg/ml))** | | | | |
|---|---|---|---|---|---|---|
| **Species** | **Strain Source** | **10 (mg/ml) MSG** | **20 (mg/ml) MSG** | **30 (mg/ml) MSG** | **40 (mg/ml) MSG** | **50 (mg/ml) MSG** |
| *Lactobacillus brevis* DPC6108 | Infant faeces | 11.01+/-0.31 | 20.47+/-0.13 | 28.02+/-0.23 | 30.39+/-0.56 | 32.32+/-1.24 |
| *Bifidobacterium adolescentis* DPC6044 | Infant faeces | 2.2+/-0.43 | 3.17+/-0.39 | 2.76+/-0.37 | 1.24+/-0.1 | 3.07+/-0.26 |
| *Bifidobacterium dentium* DPC6333 | Infant faeces | 5.25+/-0.33 | 7.69+/-0.75 | 8.62+/-0.86 | 5.54+/-0.28 | 6.16+/-0.47 |
| *Bifidobacterium dentium* NFBC2243 | Dental carries | 6.09+/-0.22 | 12.32+/-0.69 | 12.48+/-0.60 | 5.68+/-0.03 | 8.63+/-0.70 |
| *Bifidobacterium infantis* UCC35624 | Ileal-caecal region | 3.46+/-0.05 | 3.26+/-0.13 | 2.63+/-0.223 | 5.68+/-0.146 | 2.04+/-0.144 |

### γ-Amino butyric acid production by Lactobacillus brevis DPC6108 in the presence of 30 mg/ml monosodium glutamate:

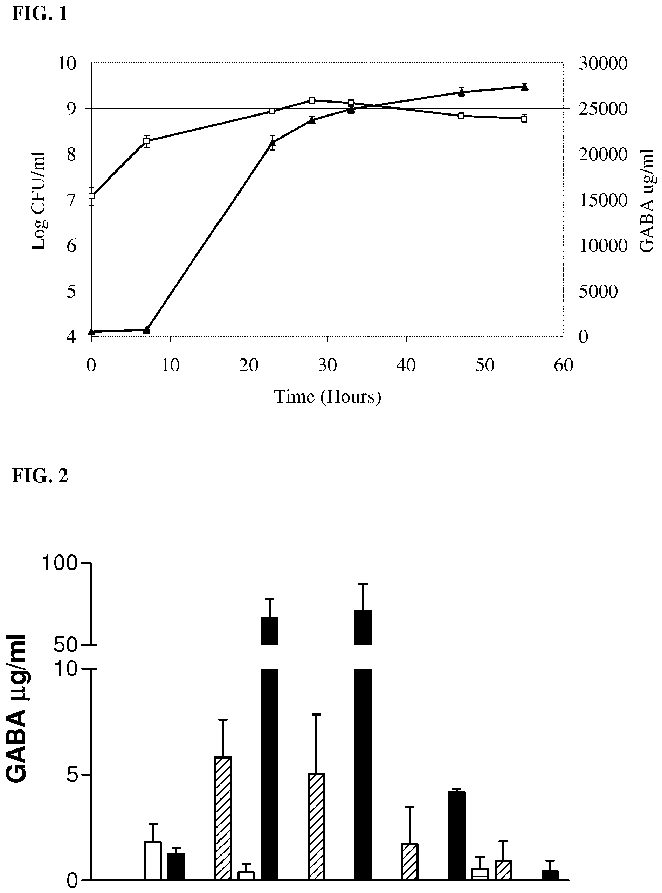

Lactobacillus brevis DPC6108 was the most efficient strain tested for the conversion of MSG to GABA. The growth of Lb. brevis DPC6108 in mMRS with 30 mg/ml was monitored over time (Fig. 1). Production of GABA was associated the stationary phase of growth of the culture. The maximum cell count was achieved at 28 h and maximum conversion of MSG to GABA was at 55 h (Fig 1).

### γ-Amino butyric acid production by the culturable gut microbiota and Lactobacillus brevis DPC6108 in the presence of 30 mg/ml monosodium glutamate:

The effect of 30 mg/ml MSG and ∼ 10⁸ live L. brevis DPC6108 on the culturable gut microbiota was investigated using a simple pH-controlled anaerobic faeces-based fermentation (Fooks and Gibson 2003). Stool samples were obtained from three adult subjects and faecal fermentations were carried out in duplicate with each sample. Samples were withdrawn at 0, 4, 9, 21 and 24 h for GABA analysis. GABA was not detected in the faecal fermentation using the sample collected from subject number 1 at 0, 4, 9 or 21 h. It was however detected at 24 h, at a concentration of 0.56µg/ml of faecal fermentation (Fig 2). GABA was detected in a faecal based fermentation using a sample collected from subject number 2. The GABA concentration reached a maximum at 4 h and subsequently decreased at 9, 21 and 24 h, with concentrations of 5.82, 5.03, 1.75 and 0.94 µg/ml, respectively (Fig 2). GABA was also detected in a faecal-based fermentation using a sample collected from subject number 3. The maximum GABA concentration of 1.83 µg/ml was recorded at 0 h and the GABA concentration subsequently decreased at 4, 9, 21 and 24 h, with concentrations of 0.39, 0, 0 and 0 µg/ml, respectively (Fig 2). Lb. brevis DPC6108 was added to a faecal-based fermentation to examine the effect of adding a potential probiotic GABA producing strain on a faecal fermentation at physiological pH. The faecal sample used to make the faecal slurry was obtained from subject 3. The GABA concentration increased to 66.25 µg/ml after 4 h and reached a maximum at 9h of 70.72 µg/ml (Fig 2). Similar to other faecal-based fermentations, the GABA concentration decreased at 21 and 24 h to 4.18 and 0.47 µg/ml, respectively (Fig 2).

### Animal Studies:

No differences in body weight gain, adrenal gland weight, serum glucose levels, or cholesterol content of livers were recorded during the study.

Behaviour tests including the Open Field test, as indicator of Anxiety, were undertaken at end of the trial. No differences in total distance moved by the animals were found, but significantly (p>0.05) higher time was spent by the animals in the GABA group in the inner zone, and data approaching significance were recorded for the *L. brevis* DPC 6108 group, compared to placebo control group, suggesting an anxiolytic-like effect in the former two groups, compared with the latter group. (A p-value of 0.0522 was obtained for the L.brevis treated group in the amount of time spent in the inner zone.).

The results of the Forced Swim Test (FST) were as follows: The pre swim results demonstrate no differences between any of the groups in immobility, swimming or climbing. The forced swim test on day 2 revealed significant increase in immobility in the GABA treated group compared to control animals indicating depressant-like activity.

## Claims

1. An isolated bacteria *Lactobacillus brevis* (DPC6108) deposited with the National Collection of Industrial and Marine Bacteria Limited (NCIMB) on 28 November 2011 and accorded the accession number NCIMB 41903, the bacteria being **characterised in that** it is capable of producing γ-aminobutyric acid (GABA).

2. An isolated bacteria of Claim 1, that is capable of 100% conversion of monosodium glutamate (MSG) to γ-aminobutyric acid (GABA) in an *in-vitro* conversion test.

3. An isolated bacteria of Claim 1, that is derived from a human neonatal faecal sample.

4. An isolated bacteria of Claim 1 or 2, in the form of live cells, dead cells, cellular components, cell extracts, or cell lysates.

5. An isolated bacteria of any preceding Claim **characterised by** a 16s rRNA sequence of SEQUENCE ID NO: 1.

6. A composition comprising an isolated bacteria of any preceding Claim.

7. A composition of Claim 6 in the form of a product formulated for human ingestion.

8. A composition according to Claim 7 in the form of a food product, a drink, a food supplement, or a medicament.

9. A composition according to any of Claims 6 to 8 and further including at least one bacterial culture selected from the group consisting of:
- *Bifidobacterium dentium* (DPC6333) NCIMB 41904 deposited on 28 November 2011;
- *Bifidobacterioum adolescentis* (DPC6044) NCIMB 41908 deposited on 2 December 2011;
- *Bifidobacterium infantis* (UCC35624) NCIMB 41003 deposited on 13 January 1999; and
- *Bifidobacterium dentium NCFB2243.*

10. A composition according to any of Claims 6 to 9 and comprising a source of glutamate.

11. A composition according to Claim 10 in which the source of glutamate is a glutamate salt.

12. A composition according to any of Claims 6 to 11 for use as a medicament.

13. A composition according to any of Claims 6 to 11 **for** use in a method of regulation or treatment of cardiovascular function, growth hormone secretion, mood disturbance, anxiety, stress, pain, renal failure, or proliferative conditions.

14. A composition for use of Claim 13 in which the composition is administered to an individual orally and forms part of the individuals' microbiota.

15. A composition for use of Claim 13 or 14 in which the composition includes free glutamate.

## Patentansprüche

1. Isoliertes Bakterium *Lactobacillus brevis* (DPC6108), das bei der National Collection of Industrial and Marine Bacteria Limited (NCIMB) am 28. November 2011 hinterlegt wurde und die Zugangsnummer NCIMB 41903 erhielt, wobei das Bakterium **dadurch gekennzeichnet ist, dass** es γ-Aminobuttersäure (GABA) produzieren kann.

2. Isoliertes Bakterium nach Anspruch 1, das Mononatriumglutamat (MSG) zu γ-Aminobuttersäure (GABA) in einem *in-vitro*-Umwandlungstest zu 100 % umwandeln kann.

3. Isoliertes Bakterium nach Anspruch 1, das von einer menschlichen neonatalen Stuhlprobe stammt.

4. Isoliertes Bakterium nach Anspruch 1 oder 2 in Form von lebenden Zellen, toten Zellen, zellulären Bestandteilen, Zellextrakten oder Zelllysaten.

5. Isoliertes Bakterium nach einem vorstehenden Anspruch, das durch eine 16S-rRNA-Sequenz von SEQUENZ-ID NR. 1 gekennzeichnet ist.

6. Zusammensetzung, die ein isoliertes Bakterium nach einem vorstehenden Anspruch umfasst.

7. Zusammensetzung nach Anspruch 6 in Form eines Produkts, das für die menschliche Einnahme formuliert ist.

8. Zusammensetzung nach Anspruch 7 in Form eines Nahrungsmittelprodukts, eines Getränks, eines Nahrungsergänzungsmittels oder eines Medikaments.

9. Zusammensetzung nach einem der Ansprüche 6 bis 8, und die weiter mindestens eine Bakterienkultur einschließt, die aus der Gruppe ausgewählt ist, die aus Folgenden besteht:
- *Bifidobacterium dentium* (DPC6333), NCIMB 41904, hinterlegt am 28. November 2011;
- *Bifidobacterium adolescentis* (DPC6044), NCIMB 41908, hinterlegt am 2. Dezember 2011;
- *Bifidobacterium infantis* (UCC35624), NCIMB 41003, hinterlegt am 13. Januar 1999; und
- *Bifidobacterium dentium NCFB2243.*

10. Zusammensetzung nach einem der Ansprüche 6 bis 9, und die eine Quelle des Glutamats umfasst.

11. Zusammensetzung nach Anspruch 10, in der die Quelle des Glutamats ein Glutamatsalz ist.

12. Zusammensetzung nach einem der Ansprüche 6 bis 11 für die Verwendung als Medikament.

13. Zusammensetzung nach einem der Ansprüche 6 bis 11 für die Verwendung in einem Verfahren zur Regulierung oder Behandlung von Herz-Kreislauf-Funktion, Sekretion von Wachstumshormon, Stimmungsstörung, Angstzustand, Stress, Schmerz, Nierenversagen oder proliferativen Leiden.

14. Zusammensetzung für die Verwendung nach Anspruch 13, in der die Zusammensetzung an ein Individuum oral verabreicht wird und Teil der Mikrobiota des Individuums bildet.

15. Zusammensetzung für die Verwendung nach Anspruch 13 oder 14, in der die Zusammensetzung freies Glutamat einschließt.

## Revendications

1. Bactérie isolée de *Lactobacillus brevis* (DPC6108) déposée auprès de la National Collection of Industrial and Marine Bacteria Limited (NCIMB) le 28 novembre 2011 et selon le numéro d'accès NCIMB 41903, la bactérie étant **caractérisée en ce qu'**elle est capable de produire de l'acide γ-aminobutyrique (GABA).

2. Bactérie isolée selon la revendication 1, capable d'une conversion à 100% de glutamate monosodique (MSG) en acide γ-aminobutyrique (GABA) dans un test de conversion *in vitro.*

3. Bactérie isolée selon la revendication 1, qui est dérivée d'un échantillon fécal néonatal humain.

4. Bactérie isolée selon la revendication 1 ou 2, sous la forme de cellules vivantes, de cellules mortes, de composants cellulaires, d'extraits cellulaires, ou de lysats cellulaires.

5. Bactérie isolée selon l'une quelconque des revendications précédentes, **caractérisée par** une séquence d'ARNr 16S de SEQUENCE ID n° 1.

6. Composition comprenant une bactérie isolée selon l'une quelconque des revendications précédentes.

7. Composition selon la revendication 6, sous la forme d'un produit formulé pour une ingestion par l'homme.

8. Composition selon la revendication 7, sous la forme d'un produit alimentaire, d'une boisson, d'un complément alimentaire, ou d'un médicament.

9. Composition selon l'une quelconque des revendications 6 à 8, comportant en outre au moins une culture bactérienne choisie dans le groupe constitué par :
- *Bifidobacterium dentium* (DPC6333) NCIMB 41904 déposée le 28 novembre 2011 ;
- *Bifidobacterium adolescentis* (DPC6044) NCIMB 41908 déposée le 2 décembre 2011 ;
- *Bifidobacterium infantis* (UCC35624) NCIMB 41003 déposée le 13 janvier 1999 ; et
- *Bifidobacterium dentium* NCFB2243.

10. Composition selon l'une quelconque des revendications 6 à 9, comprenant une source de glutamate.

11. Composition selon la revendication 10, dans laquelle la source de glutamate est un sel de glutamate.

12. Composition selon l'une quelconque des revendications 6 à 11, pour une utilisation comme médicament.

13. Composition selon l'une quelconque des revendications 6 à 11, pour une utilisation dans une méthode de régulation ou de traitement d'une fonction cardiovasculaire, de la sécrétion d'hormone de croissance, des troubles de l'humeur, de l'anxiété, du stress, des douleurs, de l'insuffisance rénale, ou de conditions prolifératives.

14. Composition pour une utilisation selon la revendication 13, où la composition est administrée à un individu par voie orale et fait partie du microbiote de l'individu.

15. Composition pour une utilisation selon la revendication 13 ou 14, où la composition comporte du glutamate libre.


### SEQUENCE LISTING

<110> University College Cork - National University of Ireland, Cork Agriculture and Food Development Authority (Teagasc)
<120> GABA-producing culturable bacteria derived from the human gastrointestinal tract
<130> P10763PC00
<150> EP 12151768.4
   <151> 2012-01-19
<160> 1
<170> PatentIn version 3.5
<210> 1
   <211> 1396
   <212> DNA
   <213> Lactobacillus brevis
<220>
   <221> misc_feature
   <223> "n" at positions 1, 23 adn 1390 is a, c, g or t
<220>
   <221> misc_feature
   <222> (1)..(1)
   <223> n is a, c, g, or t
<220>
   <221> misc_feature
   <222> (23)..(23)
   <223> n is a, c, g, or t
<220>
   <221> misc_feature
   <222> (1390)..(1390)
   <223> n is a, c, g, or t
<400> 1
   natgacgtgc ttgcactgat ttnaacaatg aagcgagtgg cgaactggtg agtaacacgt      60
   gggaaatctg cccagaagca ggggataaca cttggaaaca ggtgctaata ccgtataaca     120
   acaaaatccg catggatttt gtttgaaagg tggcttcggc tatcacttct ggatgatccc     180
   gcggcgtatt agttagttgg tgaggtaaag gcccaccaag acgatgatac gtagccgacc     240
   tgagagggta atcggccaca ttgggactga gacacggccc aaaytcctac gggaggcagc     300
   agtagggaat cttccacaat ggacgaaagt ctgatggagc aatgccgcgt gagtgaagaa     360
   gggtttcggc tcgtaaaact ctgttgttaa agaagaacac ctttgagagt aactgttcaa     420
   gggttgacgg tatttaacca gaaagccacg gctaactacg tgccagcagc cgcggtaata     480
   cgtaggtggc aagcgttgtc cggatttatt gggcgtaaag cgagcgcagg cggtttttta     540
   agtctgatgt gaaagccttc ggcttaaccg gagaagtgca tcggaaactg ggagacttga     600
   gtgcagaaga ggacagtgga actccatgtg tagcggtgga atgcgtagat atatggaaga       660
   acaccagtgg cgaaggcggc tgtctagtct gtaactgacg ctgaggctcg aaagcatggg       720
   tagcgaacag gattagatac cctggtagtc catgccgtaa acgatgagtg ctaagtgttg       780
   gagggtttcc gcccttcagt gctgcagcta acgcattaag cactccgcct ggggagtacg       840
   accgcaaggt tgaaactcaa aggaattgac gggggcccgc acaagcggtg gagcatgtgg       900
   tttaattcga agctacgcga agaaccttac caggtcttga catcttctgc caatcttaga       960
   gataagacgt tcccttcggg gacagaatga caggtggtgc atggttgtcg tcagctcgtg      1020
   tcgtgagatg ttgggttaag tcccgcaacg agcgcaaccc ttattatcag ttgccagcat      1080
   tcagttgggc actctggtga gactgccggt gacaaaccgg argaaggtgg ggatgacgtc      1140
   aaatcatcat gccccttatg acctgggcta ccaccgtgct acaatggacg gtacaacgag      1200
   tcgcgaagtc gtgaggctaa gctaatctct taaagccgtt ctcagttcgg attgtaggct      1260
   gcaactcgcc tacatgaagt tggaatcgct agtaatcgcg gatcagcatg ccgcggtgaa      1320
   tacgttcccg ggccttgtac acaccgcccg tcacaccatg agagtttgta acacccaaag      1380
   ccggtgagan acctcg                                                      1396